# EUROPÄISCHE PATENTSCHRIFT

(11) **EP 1 818 019 B1**
(45) Veröffentlichungstag und Bekanntmachung des Hinweises auf die Patenterteilung: **22.07.2009**
(21) Anmeldenummer: 07002701.6
(22) Anmeldetag: 08.02.2007
(51) Int. Cl.: A61B 17/04, A61B 17/06

(54) **Verpackung für ein medizinisches Ankerelement samt Faden**
Packaging for a medical anchor element with thread
Emballage pour un élément d'ancrage médical avec fil en velours

(30) Priorität: 10.02.2006 DE 102006007263
(43) Veröffentlichungstag der Anmeldung: 15.08.2007
(73) Patentinhaber: Karl Storz GmbH & Co. KG, 78532 Tuttlingen (DE)
(72) Erfinder: Ruffieux, Kurt, Dr., 8800 Thalwil (CH); Reichardt, Dennis, 8055 Zürich (CH); Kern, Heiner, 8472 Seuzach (CH); Siegrist, Alexander, 8907 Wettswil a.A. (CH)
(74) Vertreter: Weller, Wolfgang

(56) Entgegenhaltungen:
- EP-A1- 0 717 958
- US-A- 5 174 087
- US-A- 5 715 942
- US-A- 5 954 748
- US-A- 6 080 184

## Beschreibung

Die Erfindung betrifft eine Verpackung für ein Ankerelement und einen Faden, das samt Faden in einen Knochen zum Fixieren einer Sehne einbringbar ist und einen etwa zylindrischen Körper aufweist, mit einer äußeren Umhüllung, mit einer innerhalb der Umhüllung angeordneten Halterung, in der ein Ankerelement aufgenommen ist, und mit einer Aufnahme für den auf dem Ankerelement bereits aufgefädelten Faden, wobei die Halterung als Zuschnitt ausgebildet ist, der zumindest eine Lasche aufweist, in die das Ankerelement eingesteckt ist und die Lasche eine Sollbruchstelle aufweist.

Eine derartige Verpackung ist aus der US 5,174,087 bekannt und gemäß der Oberbegriff des Anspruchs 1.

Derartige Ankerelemente, auch Fadenanker genannt werden im medizinischen Bereich dazu eingesetzt, um von einem Knochen abgerissenes Gewebe, meist Sehnen, wieder am Knochen zu fixieren. Dazu wird das Ankerelement, in das ein Faden eingefädelt ist, in den Knochen eingetrieben. Die beiden Fadenenden werden dazu verwendet, mit der abgerissenen Sehne verknotet zu werden und diese dadurch wieder am Knochen zu fixieren.

Bei den Ankerelementen sind verschiedene Ausgestaltungen bekannt, wie sie beispielsweise aus der US 4,632,100, der US 5,690,676 und der US 6,139,565 bekannt sind.

Allgemein weist ein solches Ankerelement einen etwa zylindrischen oder stiftartigen Körper auf, an dessen Außenseite widerhakenartige Vorsprünge vorhanden sind, die dafür sorgen, dass nach Eintreiben des Ankerelements in den Knochen dieses gegen Abziehen durch die Widerhaken gehindert ist. Diese Widerhaken können auch als ein Außengewinde ausgestaltet sein, je nach der Technik des Einbringens des Ankerelementes. Eine Technik besteht darin, im Knochen eine Bohrung vorzusehen, in die das Ankerelement eingeschlagen bzw. eingetrieben wird. Eine andere Technik besteht darin, das Ankerelement ohne Vorsehen einer vorherigen Bohrung in Art einer Schraube in den Knochen bzw. die Spongiosa einzudrehen. Je nach Ausgestaltung kann das Ankerelement aus metallischem Material, beispielsweise aus Titan, hergestellt sein, es sind auch Ausgestaltungen aus bioresorbierbaren Materialien bekannt.

All diesen Ankerelementen ist gemein, dass sie zum Setzen mit einem Werkzeug verbunden werden müssen, sei es mit einem Art Schraubendreher oder mit einem sog. Einschläger.

Dazu ist üblicherweise am proximalen Ende des Ankerelements eine Einsenkung, beispielsweise in Form eines Sechsecks, vorgesehen, in die ein entsprechend geformtes Werkzeug eingeschoben wird, um das Ankerelement in den Knochen zu setzen.

Da derartige Ankerelemente relativ klein sind, dabei sind Längen im Bereich von 1 bis 1,5 cm und Durchmesser im Bereich von 3 bis 5 mm üblich, verlangt dieses Ansetzen an das Werkzeug eine gewisse Aufmerksamkeit und Fingerfertigkeit.

Zusätzlich ist das Ankerelement auf einen Faden aufgefädelt, wozu das Ankerelement in seinem Körper eine quer durchlaufende Bohrung oder am proximalen Ende entsprechende Fadenösen aufweist. Auch das Durchfädeln eines Fadens durch einen solchen kleinen Körper erfordert eine gewisse Aufmerksamkeit und Fingerfertigkeit.

Da sowohl Ankerelement als auch Faden nach einem Eingriff im Körper verbleiben, müssen diese Bauelemente steril sein, um Infektionen zu vermeiden. Bei der eigentlichen Operation wird, meist von einem Chirurgieassistenten, der Zusammenbau aus Werkzeug, Anker und Faden bewerkstelligt. Dazu wird, wie zuvor erwähnt, das Werkzeug in das proximale Ende des Ankerelements eingeschoben. Der Faden, der entweder schon vorher eingefädelt wurde oder in diesem Zustand aufgefädelt wird, wird längs des Werkzeugs nach proximal geführt und dort an radial vorstehenden Stiften befestigt, damit der Faden nicht herumbaumelt und den Setzvorgang stört.

Bei der eingangs erwähnten US 5,174,087 wird dies dadurch vereinfacht, dass die Verpackung für das Ankerelement und den Faden eine Halterung aufweist, in der ein Ankerelement mit einem auf diesen bereits aufgefädelten Faden aufgenommen ist.

Die Halterung ist dabei als Zuschnitt ausgebildet, der aus einem flächigen Material wie z.B. einem Papiermaterial ausgeschnitten oder ausgestanzt werden kann und der zumindest eine Lasche mit einer Sollbruchstelle aufweist, in die das Ankerelement eingesteckt ist und in dessen Innenraum der Faden aufgenommen ist.

Solch ein Zusammenbau aus Halterung, Ankerelement und Faden ist als steriler Zusammenbau herstellbar, der anschließend mit einer äußeren Umhüllung versehen wird, die diesen sterilen Zusammenbau zur Außenseite hin abschließt.

Soll nun unter sterilen Bedingungen ein zuvor erwähntes Werkzeug mit dem Ankerelement verbunden werden, ist es am praktischsten, wenn das Ankerelement durch die Lasche gehalten wird, um weiter unnötige Kontakte, die zu einer Unsterilität führen könnten, zu vermeiden.

Dies kann sich bei dem eingangs erwähnten Bespiel aber als problematisch darstellen, da das Ankerelement aufgrund eines zu geringen und zu lockeren Halts aus der Lasche herausrutschen kann, ohne korrekt am Werkzeug befestigt zu sein.

Es ist daher die Aufgabe der vorliegenden Erfindung, diese Verpackungen dahingehend weiterzuentwickeln, dass ein guter und sicherer Halt des Ankerelements in der Lasche gewährt ist.

Erfindungsgemäß wird die Aufgabe dadurch gelöst, dass der etwa zylindrische Körper des Ankerelements an einer Außenseite mit Haken versehen ist, die als Ringflansche ausgebildet sind, wobei der Abstand zwischen zwei Haken in etwa der Breite der Lasche entspricht.

Diese Maßnahmen haben den Vorteil, dass die Haken einen sicheren Halt des Ankerelements in der Lasche gewährleisten, da diese im Wesentlichen passgenau zwischen den Haken zu liegen kommt. Somit wird einerseits ein unerwünschtes Herausfallen, versehentliches Abziehen oder Verschieben verhindert und folglich ein besonders sicherer Halter des Ankerelements in der Halterung gewährleistet. Weiterhin ist durch die Sollbruchstelle aber auch sichergestellt, dass das Ankerelement von der Halterung abgenommen werden kann, ohne dass die Gefahr besteht, dass Relativverschiebungen zwischen Ankerelement und Werkzeug bei diesem Entnahmevorgang erfolgen.

In einer weiteren Ausgestaltung der Erfindung ist das Ankerelement lagedefiniert in der Halterung aufgenommen.

Diese Maßnahme hat den Vorteil, dass die Handhabungsperson genau weiß, in welcher Ausrichtung das Ankerelement in der Halterung aufgenommen ist, was die späteren Manipulations- und Handhabungsvorgänge sehr erleichtert.

In einer weiteren Ausgestaltung ist das Ankerelement derart in der Halterung aufgenommen, dass ein Werkzeug zur Handhabung an das Ankerelement anbringbar ist.

Diese Maßnahme hat den Vorteil, dass nicht nur eine lagedefinierte Halterung des Ankerelementes möglich ist, sondern zugleich eine zielgerechte Ausrichtung dahingehend, dass das Werkzeug noch bei in der Halterung aufgenommenem Ankerelement an dieses herangeführt und je nach Ausgestaltung an dieses angesetzt, eingeschoben oder aufgesetzt werden kann. Diese Vorgänge sind somit nicht nur vereinfacht, sondern können auch steril durchgeführt werden, ohne dass es also notwendig ist, bei diesen Manipulationen das Ankerelement zu erfassen.

In einer weiteren Ausgestaltung der Erfindung ist die Halterung als Handhabe ausgebildet.

Diese Maßnahme hat nun den erheblichen Vorteil, dass zur Erleichterung der zuvor erwähnten Vorgänge des Ansetzens des Werkzeuges an dem Ankerelement die Halterung von einer Hand der Handhabungsperson ergriffen werden und in die entsprechende günstige Ausrichtung gebracht werden kann, um das mit der anderen Hand gehaltene Werkzeug anzusetzen. Diese Kombination erleichtert die Handhabung ganz besonders und erlaubt diese Vorgänge steril durchzuführen.

In einer weiteren Ausgestaltung der Erfindung trägt die Halterung auch zugleich den Faden.

Diese Maßnahme hat den Vorteil, dass eine sehr kompakte Bauweise des Zusammenbaus entsteht, da die Halterung auch gleichzeitig als Aufnahme für den Faden dient. So kann dieser Zusammenbau aus Halterung, Faden und Ankerelement entsprechend gehandhabt werden, ohne dass dazu Relativverschiebungen zwischen dem Ankerelement und Faden stattfinden müssen. Erst nach Einschieben oder Ansetzen des Werkzeuges an das Ankerelement braucht der Faden bewegt zu werden, um ihn am Werkzeug zu fixieren.

In einer weiteren Ausgestaltung der Erfindung sind Ankerelement und Halterung unverlierbar, jedoch lösbar, miteinander verbunden.

Diese Maßnahme hat den Vorteil, dass das Ankerelement unverlierbar in der Halterung aufgenommen ist, so dass bei den zuvor beschriebenen Handhabung nicht die Gefahr besteht, dass sich das Ankerelement versehentlich von der Halterung löst, beispielsweise von dieser abfällt. Durch Vorsehen der Lösbarkeit kann, nachdem das Werkzeug an das Ankerelement angesetzt wurde und in dieses eingeschoben ist, dieser Verbund gelöst werden.

In einer weiteren Ausgestaltung der Erfindung ist der Zuschnitt zu einem umschlagartigen Körper gefaltet, in dessen Innenraum der Faden aufgenommen ist.

Diese Maßnahme hat den Vorteil, dass in dem Umschlag der relativ lange Faden als mehr oder weniger geordnetes Bündel oder Knäuel aufgenommen werden kann. Dazu kann der Faden beim Herstellen der Verpackung auf eine Zuschnittfläche gelegt werden und dann ein anderer Abschnitt darum zu einem briefumschlagartigen Körper gefaltet werden. Zum Entnehmen des Fadens ist dieser Umschlag wieder einfach zu öffnen und der Faden kann problemlos entnommen werden.

Besonders einfach wird die Lasche dadurch hergestellt, dass entsprechende Stanzungen in dem Zuschnitt bewerkstelligt werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger Ausführungsbeispiele in Zusammenhang mit den beiliegenden Zeichnungen näher beschrieben und erläutert. Es zeigen:
- Fig. 1: eine Seitenansicht eines ersten Ausführungsbeispiels einer erfindungsgemäßen Verpackung, und
- Fig. 2: eine teilweise perspektivische Ansicht der Verpackung von Fig. 1 nach Entfernen der äußeren Umhüllung während eines Abschnittes der Handhabung.

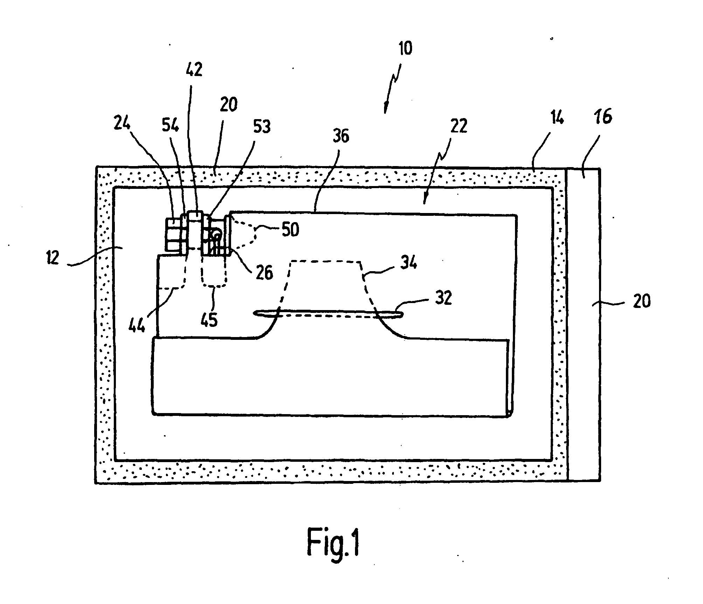

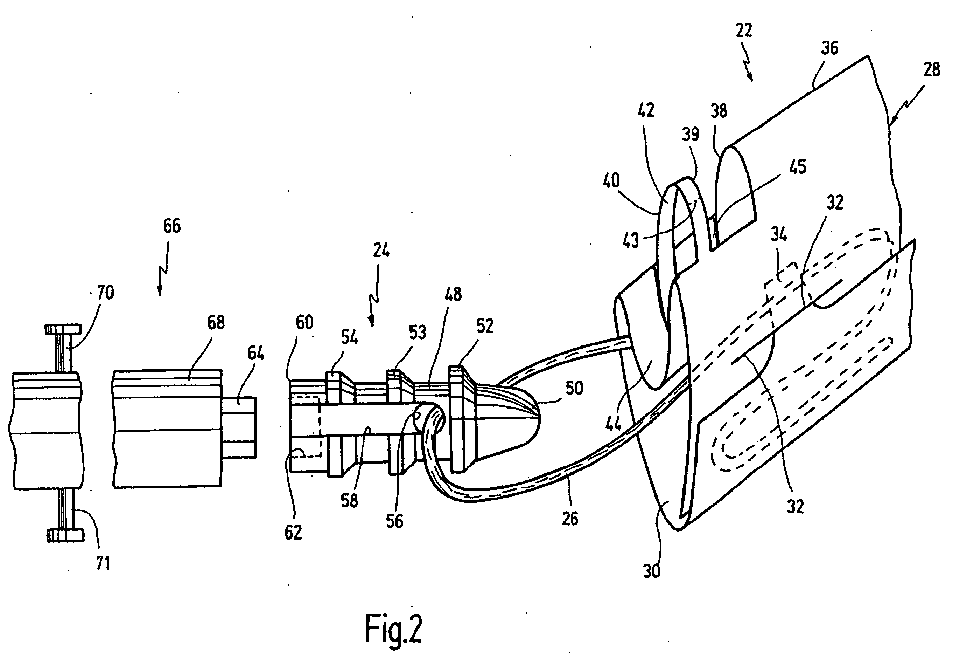

In den Fig. 1 und 2 ist ein Ausführungsbeispiel einer erfindungsgemäßen Verpackung insgesamt mit der Bezugsziffer 10 bezeichnet.

Die Verpackung 10 weist eine äußere Umhüllung 12 auf, die aus zwei übereinander gelegenen rechteckigen Folien 14 und 16 zu einer Tasche dadurch aufgebaut ist, dass die beiden Folien 14, 16 durch eine umlaufende Verklebung 18 miteinander verbunden sind.

Die rechteckige Folie 16 steht auf einer der kurzen Rechteckseiten etwas über die andere Folie 14 hinaus, wodurch eine überstehende Lasche 20 gebildet ist, die ein Aufreißen der äußeren Umhüllung 12 erleichtert. Dazu ist die Verklebung 18 als eine Haftklebung ausgebildet, die die beiden Folien 14 und 16 zu einem hermetisch zur Außenseite abgedichteten Umhüllung verbinden, dennoch ein Abziehen der beiden Folien 14 und 16 voneinander zum Öffnen ermöglicht. Die Folien selbst sind aus kunststoffbeschichteten Alufolien hergestellt.

Im Innern der äußeren Umhüllung 12 ist eine Halterung 22 aufgenommen, die ein Ankerelement 24 und einen mit dem Ankerelement 24 verbundenen Faden 26 trägt.

Die Halterung 22 ist aus einem Zuschnitt 28 aus Papier gebildet, der so gestanzt bzw. gefaltet ist, dass ein Umschlag 30 in einer Art eines Briefumschlages entsteht.

Dazu ist in einer Seite des Zuschnitts 28 ein Schlitz 32 eingestanzt, in den eine Einstecklasche 34 eingesteckt werden kann, wodurch der zuvor erwähnte Umschlag 30 gebildet wird.

Im Bereich der in Darstellung von Fig. 1 und 2 oberen Falzkante 36 sind drei Stanzschnitte 38, 39 und 40 ausgebildet, durch die eine Lasche 42 entsteht, bei der beidseits das Papiermaterial nach innen in Form von Taschen 44 und 45 eingedrückt ist. Die Lasche 42 ist, wie das insbesondere aus Fig. 2 ersichtlich ist, mit einer Sollbruchstelle 43 versehen, die gleich beim Stanzvorgang angebracht werden kann.

Wie insbesondere aus Fig. 1 ersichtlich ist, dienen diese konstruktiven Maßnahmen dazu, um das Ankerelement 24 in einer bestimmten Lage zu haltern.

Wie insbesondere aus Fig. 2 ersichtlich, weist das Ankerelement 24 einen etwa zylindrischen Körper 48 auf, der am distalen Ende in eine Spitze 50 übergeht.

An seiner Außenseite ist der Körper 48 mit drei Haken 52, 53 und 54 versehen, die als Art Ringflansche ausgebildet sind, wobei diese jeweils in Richtung der Spitze 50 über eine Anschrägung in den Körper 48 übergehen. Am gegenüberliegenden Ende gehen die Haken 42 über eine relativ eckige Hinterschneidung bzw. Stufe in den Körper 48 über. Im Bereich zwischen dem Haken 52 und 53 ist eine durch den Körper 58 durchgehende Queröffnung 56 vorgesehen, durch die ein Faden 26 hindurchgefädelt ist, und zwar so, dass beidseits der Öffnung 26 etwa gleich lange Fadenhälften zu liegen kommen. In der Außenseite des Körpers 28 ist ausgehend von der Öffnung 56 bis zum proximalen Ende 60 diametral gegenüberliegend jeweils ein Einschnitt 58 vorgesehen, der sich bis zum proximalen Ende 60 erstreckt und dort öffnet. Diese Einschnitte 58 dienen dazu, um den Faden in den Einschnitten 58 längs des Körpers 48 von der Öffnung 56 nach proximal zu führen.

Im Bereich des Endes 60 ist im Körper 48 eine Einsenkung 62 vorgesehen, beispielsweise mit sechseckförmigem Querschnitt.

Diese Einsenkung 62 dient dazu, um einen entsprechend ausgeformten Vorsprung 64 eines Werkzeuges 66, hier ein Eintreiber bzw. einen Einschläger, aufzunehmen. Das heißt, der Vorsprung 64 weist einen entsprechenden sechseckförmigen Querschnitt auf, der dem der Einsenkung 62 entspricht.

Das Werkzeug 66 ist als stabförmiger Körper 68 ausgebildet mit einem hier nicht näher bezeichneten Schaft und einem Handgriff, wobei am stabförmigen Körper 68 in axialem Abstand vom Vorsprung 64, beispielsweise im Abstand von 10 bis 15 cm, radial zwei Zapfen 70 und 71 vorstehen.

Diese Zapfen sind dazu vorgesehen, um die beiden Enden des Fadens darum zu wickeln, nachdem der Vorsprung 64 in die Einsenkung 62 eingeschoben worden ist.

In Fig. 2 ist lediglich zu Erläuterungszwecken der Konstruktion des Ankerelements 24 dieses bereits von der Halterung 22 abgenommen dargestellt, u.a. um auch zu demonstrieren, wie die beiden Enden des Fadens 26 im Innern des zu einem Umschlag 30 gefalteten Zuschnitts 28 aufgenommen sind. Ferner ist zur Erläuterung dargestellt, dass der Vorsprung 64 noch nicht in die Einsenkung 62 eingeschoben ist.

Ferner ist aus Fig. 2 in Zusammenhang mit Fig. 1 zu entnehmen, dass der Abstand zwischen den beiden Haken 53 und 54 in etwa der Breite der Lasche 42 entspricht.

Aus Fig. 1 ist zu entnehmen, dass das Ankerelement 24 so seitlich in die Halterung 22 eingesteckt ist, dass die Lasche 42 zwischen diesen beiden Haken 53 und 54 zu liegen kommt. Das Einführen ist ja dadurch erleichtert, dass die Haken in dieser Richtung angeschrägt sind, ein Abziehen oder ein versehentliches Herabfallen ist durch die Hinterschneidung bzw. Schulter am proximalen Ende der Haken 53, 54 gehindert.

Beim Herstellen der Verpackung 10 wird der Zuschnitt ausgestanzt, der Faden 26, auf den bereits das Ankerelement 24 aufgefädelt ist, in den Umschlag 30 eingelegt, dieser wird dann durch Einstecken der Einstecklasche 34 in den Schlitz 32 geschlossen und das Ankerelement 24 wird in die Lasche 42 eingeschoben. Dieser Zusammenbau kann nun sterilisiert werden oder diese Bauteile können vor Zusammenfügen sterilisiert werden, wobei dann dafür Sorge getragen werden muss, dass das Falten und Einstecken entsprechend steril, beispielsweise auch automatisiert, abläuft.

Der Zusammenbau aus Halterung 22, darin steckendem Ankerelement 24 und darin aufgenommenem Faden 26 wird nun in den Innenraum der äußeren Umhüllung 12 eingeschoben und die Verklebung 18 bewerkstelligt, beispielsweise durch eine Heißverklebung oder dergleichen. In diesem in Fig. 1 dargestellten verpackten Zustand kann die Verpackung 10 nunmehr transportiert und gehandhabt werden.

Bei der Handhabung zur Vorbereitung eines chirurgischen Eingriffs wird die äußere Umhüllung 12 aufgerissen und die Halterung 22, also der Umschlag 30, aus der aufgerissenen Umhüllung entnommen. Dazu kann beispielsweise der Umschlag 30 am rechten äußeren Ende von einer Hand einer Handhabungsperson ergriffen werden.

Dadurch, dass das Ankerelement 24 ja genau lagedefiniert und so in den Umschlag 30 eingesteckt ist, dass dessen proximales Ende 60 mit seiner Einsenkung 62 am linken oberen Ende vorsteht, kann nun mit der anderen Hand der Handhabungsperson das Werkzeug 66 an der Einsenkung 62 angesetzt und der Vorsprung 64 eingeschoben werden. Durch eine seitliche Kippbewegung kann nunmehr die Sollbruchstelle 43 der Lasche 42 aufgebrochen werden und der Zusammenbau aus Werkzeug 66 und Ankerelement 24 kann vom Umschlag 30 abgezogen werden, wobei dann der bereits im Ankerelement 24 eingefädelte Faden 26 ebenfalls abgezogen wird. Um dies zu erleichtern, kann beispielsweise die Einstecklasche 34 aus dem Schlitz 34 abgezogen werden und dadurch der Umschlag 30 geöffnet werden. Anschließend werden die beiden Enden des Fadens 26 um die Zapfen 70 bzw. 71 gewickelt, wonach dann das Ankerelement 24 zum Setzen im Knochen vorbereitet ist.

## Patentansprüche

1. Verpackung mit einem Ankerelement (24) und einem Faden (26), das samt Faden (26) in einen Knochen zum Fixieren einer Sehne einbringbar ist und einen etwa zylindrischen Körper (48) aufweist, mit einer äußeren Umhüllung (12), mit einer innerhalb der Umhüllung (12) angeordneten Halterung (22), in der ein Ankerelement (24) aufgenommen ist, und mit einer Aufnahme für den auf dem Ankerelement bereits aufgefädelten Faden (26), wobei die Halterung (22) als Zuschnitt (28) ausgebildet ist, der zumindest eine Lasche (42) aufweist, in die das Ankerelement (24) eingesteckt ist und wobei die Lasche (42) eine Sollbruchstelle (43) aufweist, **dadurch gekennzeichnet, dass** der etwa zylindrische Körper (48) des Ankerelements (24) an einer Außenseite mit Haken (52, 53, 54) versehen ist, die als Ringflansche ausgebildet sind, wobei der Abstand zwischen zwei Haken (53, 54) in etwa der Breite der Lasche (42) entspricht.

2. Verpackung nach Anspruch 1, **dadurch gekennzeichnet, dass** das Ankerelement (24) lagedefiniert in der Halterung (22) aufgenommen ist.

3. Verpackung nach Anspruch 1 oder 2, **dadurch gekennzeichnet, dass** das Ankerelement (24) derart in der Halterung (22) aufgenommen ist, dass ein Werkzeug (66) zur Handhabung an das Ankerelement (24) anbringbar ist.

4. Verpackung nach einem der Ansprüche 1 bis 3, **dadurch gekennzeichnet, dass** die Halterung (22) als Handhabe ausgebildet ist.

5. Verpackung nach einem der Ansprüche 1 bis 4, **dadurch gekennzeichnet, dass** die Halterung (22) zugleich den Faden (26) trägt.

6. Verpackung nach einem der Ansprüche 1 bis 5, **dadurch gekennzeichnet, dass** Ankerelement (24) und Halterung (22) unverlierbar, jedoch lösbar, miteinander verbunden sind.

7. Verpackung nach einem der Ansprüche 1 bis 6, **dadurch gekennzeichnet, dass** der Zuschnitt (28) zu einem umschlagartigen Körper (30) gefaltet ist, in dessen Innenraum der Faden (26) aufgenommen ist.

8. Verpackung nach einem der Ansprüche 1 bis 7, **dadurch gekennzeichnet, dass** die Lasche (42) durch Stanzungen (38, 39, 40) im Zuschnitt (28) bewerkstelligt ist.

## Claims

1. Package with an anchor element (24) and a suture thread (26), which anchor element (24) can be introduced together with the suture thread (26) into a bone for the purpose of fixing a tendon and which has an approximately cylindrical body (48) with an outer casing (12), with a holder (22) which is arranged inside the casing (12) and in which an anchor element (24) is received, and with a seat for the suture thread (26) already threaded on the anchor element, said holder (22) is designed as a blank (28) which has at least one flap (42) into which the anchor element (24) is inserted, and wherein said flap (42) has a predetermined break point (43), **characterized in that** the approximately cylindrical body (48) of the anchor element (24) is provided at its outside with hooks (52, 53, 54), which are designed as annular flanges, a distance between two hooks (53, 54) corresponds approximately to the width of the flap (42).

2. Package of claim 1, **characterized in that** the anchor element (24) is received in a defined position in the holder (22).

3. Package of claims 1 or 2, **characterized in that** the anchor element (24) is received in the holder (22) in such a way that a tool (66) for handling can be applied to the anchor element (24).

4. Package of anyone of claims 1 through 3, **characterized in that** the holder (22) is designed as a handle.

5. Package of anyone of claims 1 through 4, **characterized in that** the holder (22) at the same time carries the suture thread (26).

6. Package of anyone of claims 1 through 5, **characterized in that** the anchor element (24) and the holder (22) are connected captively to one another, but in a detachable manner.

7. Package of anyone of claims 1 through 6, **characterized in that** the blank (28) is folded so as to form an envelop-like body (30), in the interior of which the suture thread (26) is received.

8. Package of anyone of claims 1 through 7, **characterized in that** the flap (42) is produced by punchies (38, 39, 40) made in the blank (28).

## Revendications

1. Emballage avec un élément d'ancrage (24) et un fil (26), l'élément d'ancrage (24) pouvant être introduit conjointement avec le fil (26) dans un os pour fixer un tendon et présentant un corps approximativement cylindrique (48), ledit emballage comprenant une housse extérieure (12), avec un support (22), disposé à l'intérieur de la housse (12) et dans lequel est reçu un élément d'ancrage (24), et avec un logement pour le fil (26) déjà enfilé sur l'élément d'ancrage, sachant que le support (22) est réalisé sous forme de pièce découpée (28) qui présente au moins une patte (42) dans laquelle est inséré l'élément d'ancrage (24) et sachant que la patte (42) présente un point de rupture privilégiée (43), **caractérisé en ce que** le corps approximativement cylindrique (48) de l'élément d'ancrage (24) est pourvu sur un côté extérieur de crochets (52, 53, 54) qui sont réalisés sous la forme de collets annulaires, sachant que la distance entre deux crochets (53, 54) correspond approximativement à la largeur de la patte (42).

2. Emballage selon la revendication 1, **caractérisé en ce que** l'élément d'ancrage (24) est reçu en position définie dans le support (22).

3. Emballage selon la revendication 1 ou 2, **caractérisé en ce que** l'élément d'ancrage (24) est reçu dans le support (22) de telle sorte qu'un outil (66) de manipulation peut être appliqué sur l'élément d'ancrage (24).

4. Emballage selon l'une des revendications 1 à 3, **caractérisé en ce que** le support (22) est réalisé sous forme d'élément de préhension.

5. Emballage selon l'une des revendications 1 à 4, **caractérisé en ce que** le support (22) porte en même temps le fil (26).

6. Emballage selon l'une des revendications 1 à 5, **caractérisé en ce que** l'élément d'ancrage (24) et le support (22) sont mutuellement reliés de manière imperdable, mais détachable.

7. Emballage selon l'une des revendications 1 à 6, **caractérisé en ce que** la pièce découpée (28) est pliée en un corps (30) du genre enveloppe, à l'intérieur de laquelle est reçu le fil (26).

8. Emballage selon l'une des revendications 1 à 7, **caractérisé en ce que** la patte (42) est réalisée par des poinçonnages (38, 39, 40) dans la pièce découpée (28).
